(19) 

**Europäisches Patentamt**
**European Patent Office**
**Office européen des brevets**

(11) **EP 4 560 013 A1**

(12) **EUROPEAN PATENT APPLICATION**
published in accordance with Art. 153(4) EPC

(43) Date of publication:
**28.05.2025 Bulletin 2025/22**

(21) Application number: **23839737.6**

(22) Date of filing: **20.02.2023**

(51) International Patent Classification (IPC):
***C12N 5/0783*** (2010.01)

(52) Cooperative Patent Classification (CPC):
**C12N 5/06**

(86) International application number:
**PCT/KR2023/002414**

(87) International publication number:
**WO 2024/014643 (18.01.2024 Gazette 2024/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.07.2022 KR 20220085308
30.01.2023 KR 20230011635**

(71) Applicant: **Novocell Bio Co., Ltd.
Incheon 22013 (KR)**

(72) Inventor: **MOON, Guy Young
Seongnam-si, Gyeonggi-do 13479 (KR)**

(74) Representative: **Weickmann & Weickmann
PartmbB
Postfach 860 820
81635 München (DE)**

(54) **METHOD FOR PRODUCING IMMUNOCYTES HAVING IMPROVED CYTOTOXICITY**

(57)  The present invention relates to a method for producing immune cells with enhanced cytotoxicity. The production method may provide immune cells with enhanced cytotoxicity, and the immune cells may be used as various cell therapy products due to their enhanced cytotoxicity.

Fig.1

NK cells
(CD3-CD56+CD16+)

EP 4 560 013 A1

**Description**

**Technical Field**

The present invention relates to a method for producing immune cells with enhanced cytotoxicity.

**Background Art**

Abnormalities and imbalances in the human immune system result in susceptibility to inflammatory reactions or the development of incurable diseases such as cancer. Weakened immunity can lead to a weakened ability to defend against viral infections or bacterial invasion, resulting in loss of health. In addition, exposure to excessive stress can also cause weakened immunity, and in particular, can cause damage and mutation of normal cells, such as the occurrence of cancer cells. To counter these changes, the human body produces immune cells (lymphocytes) every day to build a defense system, in which representative lymphocytes are natural killer cells (NK cells).

In general, NK immune cells are cultured after being activated using animal-derived antibody reagents. However, in order to produce cell therapy products that may be administered to humans, animal-derived antibody reagents should be excluded. Most antibody reagents are products that may be used for research purposes only, so they are not suitable for use in the production of cell therapy products that may be administered to humans, and there has been a continuous demand for them up to now.

Accordingly, the present inventors have found that NK cells may have better efficacy when they are activated using a pharmaceutical product produced in a GMP facility, thereby completing the present invention.

**DISCLOSURE**

**Technical Problem**

An object of one aspect of the present invention is to provide a method for producing immune cells with enhanced cytotoxicity, including: step (a) of repeating a process of adding any one or more of aldesleukin, IL-15, IL-18, anti-CD56 antibody, human immunoglobulin, and plasma to lymphocytes and culturing the lymphocytes, 1 to 7 times; step (b) of further adding any one or more of panobinostat, trichostatin A, valproic acid, sodium butyrate, and suberoylanilide hydroxamic acid (SAHA) during any one or more culturing processes in the step of repeating; and step (c) of harvesting the cultured lymphocytes.

An object of another aspect of the present invention is to provide a method for producing immune cells with enhanced cytotoxicity, including: step (a) of performing any one or more of ① addition of any two or more of aldesleukin, IL-15, IL-18, anti-CD56 antibody, human immunoglobulin, and plasma, and ② addition of any one or more of panobinostat, trichostatin A, valproic acid, sodium butyrate, and SAHA, in culture of lymphocytes; and step (b) of harvesting the lymphocytes cultured in step (a).

**Technical Solution**

One aspect of the present invention provides a method for producing immune cells with enhanced cytotoxicity, including: step (a) of repeating a process of adding any one or more of aldesleukin, IL-15, IL-18, anti-CD56 antibody, human immunoglobulin, and plasma to lymphocytes and culturing the lymphocytes, 1 to 7 times; step (b) of further adding any one or more of panobinostat, trichostatin A, valproic acid, sodium butyrate, and suberoylanilide hydroxamic acid (SAHA) during any one or more culturing processes in the step of repeating; and step (c) of harvesting the cultured lymphocytes.

In one embodiment of the present invention, step (a) may include adding any one or more of anti-CD56 antibody and human immunoglobulin and any one or more of aldesleukin, IL-15, IL-18, and plasma.

In one embodiment of the present invention, the anti-CD56 antibody may be added in an amount of 0.5 to 5 μl, and the human immunoglobulin may be added in an amount of 5 to 500 μl.

In one embodiment of the present invention, the valproic acid may be added at a concentration of 50 to 5,000 μM.

Another aspect of the present invention provides a method for producing immune cells with enhanced cytotoxicity, including: step (a) of performing any one or more of ① addition of any two or more of aldesleukin, IL-15, IL-18, anti-CD56 antibody, human immunoglobulin, and plasma, and ② addition of any one or more of panobinostat, trichostatin A, valproic acid, sodium butyrate, and SAHA, in culture of lymphocytes; and step (b) of harvesting the lymphocytes cultured in step (a).

In one embodiment of the present invention, the method for producing immune cells with enhanced cytotoxicity may further include, after step (a), step (a-1) of performing any one or more of the following: ① adding any one or more of aldesleukin, IL-15, IL-18, anti-CD56 antibody, human immunoglobulin, and plasma to the lymphocytes; and ② adding any one or more of panobinostat, trichostatin A, valproic acid, sodium butyrate, and SAHA to the lymphocytes.

In one embodiment of the present invention, the method for producing immune cells with enhanced cytotoxicity may further include, after step (a-1), step (a-2) of performing any one or more of the following: ① adding any two or more of aldesleukin, IL-15, IL-18, anti-CD56 antibody, human immunoglobulin, and plasma to the lymphocytes; and ② adding any one or more of panobinostat, trichostatin A, valproic acid, sodium butyrate, and SAHA to the lymphocytes.

In one embodiment of the present invention, the method for producing immune cells with enhanced cytotoxicity may further include, after step (a-2), step (a-3) of performing any one or more of the following: ① adding any two or more of aldesleukin, IL-15, IL-18, anti-CD56 antibody, human immunoglobulin, and plasma to the lymphocytes; and ② adding any one or more of panobinostat, trichostatin A, valproic acid, sodium butyrate, and SAHA to the lymphocytes.

In one embodiment of the present invention, the method for producing immune cells with enhanced cytotoxicity may further include, after step (a-3), step (a-4) of performing any one or more of the following: ① adding any two or more of aldesleukin, IL-15, IL-18, anti-CD56 antibody, human immunoglobulin, and plasma to the lymphocytes; and ② adding any one or more of panobinostat, trichostatin A, valproic acid, sodium butyrate, and SAHA to the lymphocytes.

In one embodiment of the present invention, the method for producing immune cells with enhanced cytotoxicity may further include, after step (a-4), step (a-5) of performing any one or more of the following: ① adding any two or more of aldesleukin, IL-15, IL-18, anti-CD56 antibody, human immunoglobulin, and plasma to the lymphocytes; and ② adding any one or more of panobinostat, trichostatin A, valproic acid, sodium butyrate, and SAHA to the lymphocytes.

In one embodiment of the present invention, the addition of any two or more of aldesleukin, IL-15, IL-18, anti-CD56 antibody, human immunoglobulin, and plasma may be treatment with any one or more of anti-CD56 antibody and human immunoglobulin and any one or more of aldesleukin, IL-15, IL-18, and plasma.

In one embodiment of the present invention, the anti-CD56 antibody may be added in an amount of 0.5 to 5 $\mu$l, and the human immunoglobulin may be added in an amount of 5 to 500 $\mu$l.

In one embodiment of the present invention, the valproic acid may be added at a concentration of 50 to 5,000 $\mu$M.

## Advantageous Effects

The production method of the present invention may provide immune cells with enhanced cytotoxicity.

## Brief Description of Drawings

FIGS. 1 to 4 show the results of analyzing the proportions of the NK and NKT cells produced by the production method according to one embodiment of the present invention.
FIGS. 5 and 6 show the results of analyzing the receptor expression levels in the NK cells produced by the production method according to one embodiment of the present invention.
FIGS. 7 to 12 show the results of tests for evaluating the cytotoxicity against cancer cells of the NK cells produced by the production method according to one embodiment of the present invention.
FIGS. 13 to 16 show the results of tests for analyzing the amount of interferon gamma (IFN-r) secreted by the NK cells produced by the production method according to one embodiment of the present invention.

## Best Mode

One aspect of the present invention provides a method for producing immune cells with enhanced cytotoxicity, including: step (a) of repeating a process of adding any one or more of aldesleukin, IL-15, IL-18, anti-CD56 antibody, human immunoglobulin, and plasma to lymphocytes and culturing the lymphocytes, 1 to 7 times; step (b) of further adding any one or more of panobinostat, trichostatin A, valproic acid, sodium butyrate, and suberoylanilide hydroxamic acid (SAHA) during any one or more culturing processes in the step of repeating; and step (c) of harvesting the cultured lymphocytes.

In the present invention, the step of repeating is a step of repeating treatment of lymphocytes with any one or more of aldesleukin, IL-15, IL-18, anti-CD56 antibody, human immunoglobulin, and plasma 1 to 7 times. Here, repeating treatment means repeating treatment with any one or more of aldesleukin, IL-15, IL-18, anti-CD56 antibody, human immunoglobulin, and plasma, and the agents used for treatment in the step of repeating may be different for each treatment step.

In the treatment with any one or more of aldesleukin, IL-15, IL-18, anti-CD56 antibody, human immunoglobulin, and plasma, the lymphocytes are treated directly with the human immunoglobulin in the culture process without coating the flask with the human immunoglobulin. This can simplify the process and shorten the coating time.

In this specification, "lymphocytes" are key cells that constitute the immune system and have the characteristics of adaptive immunity, antigen specificity, receptor diversity, memory, and self-nonself distinction. They play a central role in adaptive immunity by recognizing antigens presented by different types of white blood cells after phagocytosis of foreign substances, thereby secreting cytokines and antibodies. The lymphocytes may include NK cells.

In the present invention, the lymphocytes may be isolated and collected by centrifugation after being isolated from peripheral blood, and may be prepared by being suspended in a buffer solution or the like for additional procedures.

In one embodiment of the present invention, the treatment with any one or more of aldesleukin, IL-15, IL-18, anti-CD56 antibody, human immunoglobulin, and plasma may be performed by adding any one or more of anti-CD56 antibody and human immunoglobulin and any one or more of aldesleukin, IL-15, IL-18, and plasma. According to one embodiment of the present invention, it can be confirmed that the viability and cytotoxicity of NK cells are significantly enhanced through such treatment.

In one embodiment of the present invention, the anti-CD56 antibody may be used in an amount of 0.5 to 5 μl, and the human immunoglobulin may preferably be used in an amount of 5 to 500 μl, more preferably 5 to 50 μl. If the amounts used for treatment are outside the above ranges, the cells may not be activated or the cells may die, and thus the effect of enhancing cytotoxicity or cell viability intended by the present invention may not be obtained.

The step further including treatment with any one or more of panobinostat, trichostatin A, valproic acid, sodium butyrate, and suberoylanilide hydroxamic acid (SAHA) is a step of repeating treatment with any one or more of panobinostat, trichostatin A, valproic acid, sodium butyrate, and SAHA, which are histone deacetylase (HDAC) inhibitors, 1 to 7 times after any one of the repeated treatments. Specifically, it means further treating the lymphocytes with any one or more of panobinostat, trichostatin A, valproic acid, sodium butyrate and SAHA between repeated treatments with or after 1 to 7 repetitions of treatment with any one or more of aldesleukin, IL-15, IL-18, anti-CD56 antibody, human immunoglobulin and plasma. The number of additional treatments is not limited, but considering that the lymphocytes are additionally treated after they are treated with any one or more of aldesleukin, IL-15, IL-18, anti-CD56 antibody, human immunoglobulin, and plasma, the lymphocytes may be treated at any point between repeated treatments with any one or more of aldesleukin, IL-15, IL-18, anti-CD56 antibody, human immunoglobulin, and plasma or treated 1 to 7 times after the repeated treatments. When additional treatments are repeated, the agents used for the additional treatments may be the same or different for each treatment step.

In one embodiment of the present invention, the valproic acid may be used at a concentration of 50 to 5,000 μM for treatment. If the valproic acid is used at a concentration outside the above range, the effect of enhancing cytotoxicity or cell viability intended by the present invention may not be obtained. According to one embodiment, if the valproic acid is used at a concentration of less than 50 μM, the effect of enhancing cytotoxicity or cell viability will not occur, and if the valproic acid is used at a concentration of more than 5,000 μM, cell proliferation will not occur.

Another aspect of the present invention provides a method for producing immune cells with enhanced cytotoxicity, including: step (a) of performing any one or more of ① addition of any two or more of aldesleukin, IL-15, IL-18, anti-CD56 antibody, human immunoglobulin, and plasma, and ② addition of any one or more of panobinostat, trichostatin A, valproic acid, sodium butyrate, and SAHA, in culture of lymphocytes; and step (b) of harvesting the lymphocytes cultured in step (a).

Step (a) may include either treating the lymphocytes with any two or more of aldesleukin, IL-15, IL-18, anti-CD56 antibody, human immunoglobulin, and plasma, or treating the lymphocytes with any two or more of aldesleukin, IL-15, IL-18, anti-CD56 antibody, human immunoglobulin, and plasma, and further treating the lymphocyte with any one or more of panobinostat, trichostatin A, valproic acid, sodium butyrate, and SAHA.

In one embodiment of the present invention, the method may further include, after step (a) but before step (b), step (a-1) of performing any one or more of the following: ① adding any one or more of aldesleukin, IL-15, IL-18, anti-CD56 antibody, human immunoglobulin, and plasma to the treated lymphocytes; and ② adding any one or more of panobinostat, trichostatin A, valproic acid, sodium butyrate, and SAHA to the treated lymphocytes.

Step (a-1) may include: treating the lymphocytes, treated in step (a), with any one or more of aldesleukin, IL-15, IL-18, anti-CD56 antibody, human immunoglobulin, and plasma; or further treating the lymphocytes with any one or more of panobinostat, trichostatin A, valproic acid, sodium butyrate, and SAHA; or both. Specifically, when treatment with any two or more of aldesleukin, IL-15, IL-18, anti-CD56 antibody, human immunoglobulin, and plasma is performed in step (a), the lymphocytes may be further treated with any one or more of aldesleukin, IL-15, IL-18, anti-CD56 antibody, human immunoglobulin, and plasma, or with any one or more of panobinostat, trichostatin A, valproic acid, sodium butyrate, and SAHA, or when treatment with any two or more of aldesleukin, IL-15, IL-18, anti-CD56 antibody, human immunoglobulin, and plasma and further treatment with any one or more of panobinostat, trichostatin A, valproic acid, sodium butyrate, and SAHA are performed in step (a), the lymphocytes may be further treated with any one or more of aldesleukin, IL-15, IL-18, anti-CD56 antibody, human immunoglobulin, and plasma, and treated with any one or more of panobinostat, trichostatin A, valproic acid, sodium butyrate, and SAHA.

In one embodiment of the present invention, the method may further include, after step (a-1) but before step (b), step (a-2) of performing any one or more of the following: ① adding any two or more of aldesleukin, IL-15, IL-18, anti-CD56 antibody, human immunoglobulin, and plasma to the treated lymphocytes; and ② adding any one or more of panobinostat, trichostatin A, valproic acid, sodium butyrate, and SAHA to the treated lymphocytes.

Step (a-2) may include: treating the lymphocytes, treated in step (a-1), with any two or more of aldesleukin, IL-15, IL-18, anti-CD56 antibody, human immunoglobulin, and plasma; or further treating the lymphocytes with any one or more of panobinostat, trichostatin A, valproic acid, sodium butyrate, and SAHA; or both. Specifically, when treatment with any one or more of aldesleukin, IL-15, IL-18, anti-CD56 antibody, human immunoglobulin, and plasma is performed in step (a-1), the lymphocytes may be further treated with any two or more of aldesleukin, IL-15, IL-18, anti-CD56 antibody, human immunoglobulin, and plasma, or with any one or more of panobinostat, trichostatin A, valproic acid, sodium butyrate, and SAHA, or when treatment with any one or more of panobinostat, trichostatin A, valproic acid, sodium butyrate, and SAHA is performed in step (a-1), the lymphocytes may be further treated with any one or more of aldesleukin, IL-15, IL-18, anti-CD56 antibody, human immunoglobulin, and plasma, and treated with any one or more of panobinostat, trichostatin A, valproic acid, and sodium butyrate.

In one embodiment of the present invention, the method may further include, after step (a-2) but before step (b), step (a-3) of treating the treated lymphocytes by any one or more of the following: treatment with any one or more of aldesleukin, IL-15, IL-18, anti-CD56 antibody, human immunoglobulin, and plasma; and treatment with any one or more of panobinostat, trichostatin A, valproic acid, sodium butyrate, and SAHA.

Step (a-3) may include: treating the lymphocytes, treated in step (a-2), with any one or more of aldesleukin, IL-15, IL-18, anti-CD56 antibody, human immunoglobulin, and plasma; or further treating the lymphocytes with any one or more of panobinostat, trichostatin A, valproic acid, sodium butyrate, and SAHA; or both. Specifically, when treatment with any two or more of aldesleukin, IL-15, IL-18, anti-CD56 antibody, human immunoglobulin, and plasma is performed in step (a-2), the lymphocytes may be further treated with any one or more of aldesleukin, IL-15, IL-18, anti-CD56 antibody, human immunoglobulin, and plasma, or with any one or more of panobinostat, trichostatin A, valproic acid, sodium butyrate, and SAHA, or when treatment with any two or more of aldesleukin, IL-15, IL-18, anti-CD56 antibody, human immunoglobulin, and plasma and further treatment with any one or more of panobinostat, trichostatin A, valproic acid, sodium butyrate, and SAHA are performed in step (a-2), the lymphocytes may be further treated with any one or more of aldesleukin, IL-15, IL-18, anti-CD56 antibody, human immunoglobulin, and plasma, and treated with any one or more of panobinostat, trichostatin A, valproic acid, sodium butyrate, and SAHA.

In one embodiment of the present invention, the method may further include, after step (a-3) but before step (b), step (a-4) of treating the treated lymphocytes by any one or more of the following: treatment with any two or more of aldesleukin, IL-15, IL-18, anti-CD56 antibody, human immunoglobulin, and plasma; and treatment with any one or more of panobinostat, trichostatin A, valproic acid, sodium butyrate, and SAHA.

Step (a-4) may include: treating the lymphocytes, treated in step (a-3), with any two or more of aldesleukin, IL-15, IL-18, anti-CD56 antibody, human immunoglobulin, and plasma; or further treating the lymphocytes with any one or more of panobinostat, trichostatin A, valproic acid, sodium butyrate, and SAHA; or both. Specifically, when treatment with any one or more of aldesleukin, IL-15, IL-18, anti-CD56 antibody, human immunoglobulin, and plasma is performed in step (a-3), the lymphocytes may be further treated with any two or more of aldesleukin, IL-15, IL-18, anti-CD56 antibody, human immunoglobulin, and plasma, or with any one or more of panobinostat, trichostatin A, valproic acid, sodium butyrate, and SAHA, or when treatment with any one or more of panobinostat, trichostatin A, valproic acid, sodium butyrate, and SAHA is performed in step (a-3), the lymphocytes may be further treated with any one or more of aldesleukin, IL-15, IL-18, anti-CD56 antibody, human immunoglobulin, and plasma, and treated with any one or more of panobinostat, trichostatin A, valproic acid, sodium butyrate, and SAHA.

In one embodiment of the present invention, the method may further include, after step (a-4) but before step (b), step (a-5) of treating the treated lymphocytes by any one or more of the following: treatment with any one or more of aldesleukin, IL-15, IL-18, anti-CD56 antibody, human immunoglobulin, and plasma; and treatment with any one or more of panobinostat, trichostatin A, valproic acid, sodium butyrate, and SAHA.

Step (a-5) may include: treating the lymphocytes, treated in step (a-4), with any one or more of aldesleukin, IL-15, IL-18, anti-CD56 antibody, human immunoglobulin, and plasma; or further treating the lymphocytes with any one or more of panobinostat, trichostatin A, valproic acid, sodium butyrate, and SAHA; or both. Specifically, when treatment with any two or more of aldesleukin, IL-15, IL-18, anti-CD56 antibody, human immunoglobulin, and plasma is performed in step (a-4), the lymphocytes may be further treated with any one or more of aldesleukin, IL-15, IL-18, anti-CD56 antibody, human immunoglobulin, and plasma, or with any one or more of panobinostat, trichostatin A, valproic acid, sodium butyrate, and SAHA, or when treatment with any two or more of aldesleukin, IL-15, IL-18, anti-CD56 antibody, human immunoglobulin, and plasma and further treatment with any one or more of panobinostat, trichostatin A, valproic acid, sodium butyrate, and SAHA are performed in step (a-4), the lymphocytes may be further treated with any one or more of aldesleukin, IL-15, IL-18, anti-CD56 antibody, human immunoglobulin, and plasma, and treated with any one or more of panobinostat, trichostatin A, valproic acid, sodium butyrate, and SAHA.

In one embodiment of the present invention, the treatment with any two or more of aldesleukin, IL-15, IL-18, anti-CD56 antibody, human immunoglobulin, and plasma may be treatment with any one or more of anti-CD56 antibody and human immunoglobulin and any one or more of aldesleukin, IL-15, IL-18, and plasma. Specifically, the lymphocytes may be treated with any one or both of anti-CD56 antibody and human immunoglobulin as major components while being treated with any one or more of aldesleukin, IL-15, IL-18 and plasma. According to one embodiment of the present invention, such treatment may enhance the viability and cytotoxicity of NK cells.

In one embodiment of the present invention, the anti-CD56 antibody may be used in an amount of 0.5 to 5 $\mu$l, and the human immunoglobulin may preferably be used in an amount of 5 to 500 $\mu$l, more preferably 5 to 50 $\mu$l. In one embodiment of the present invention, the valproic acid may be used at a concentration of 50 to 5,000 $\mu$M for treatment.

In the present invention, the step of harvesting the cultured lymphocytes may be performed by a known method to harvest the cells, and specifically, only the target lymphocytes may be isolated and harvested through centrifugation. Immune cells produced by this production method have significantly enhanced cytotoxicity compared to those produced by conventional methods, and thus may be used as various cell therapy products.

**Mode for Invention**

Hereinafter, one or more embodiments will be described in more detail by way of examples. However, these examples are intended to illustrate one or more embodiments and the scope of the present invention is not limited to these examples.

**Example: Production of Immune Cells Including NK Cells with Enhanced Cytotoxicity**

Using blood samples from three different persons, immune cells including NK cells with enhanced cytotoxicity were produced through a total of nine steps as follows.

**(1) step 1 - Isolation of Lymphocytes**

Lymphocytes were isolated from human peripheral blood. Specifically, 60 to 90 ml of human peripheral blood was collected using a heparinized 10-ml vacuum tube (BD Vacutainer™). Then, 15 ml of Ficoll-Paque Plus (endotoxin tested; 1.077 g/ml density; GE Healthcare, USA) solution was placed in a 50-ml lymphocyte separation tube (Leuco sep, Greiner Bio-One, Swiss), and centrifuged at 2,000 rpm so that the solution settled down the glass membrane in the tube. The collected blood was transferred to the separation tube and centrifuged at 2,500 to 3,500 rpm for 12 to 25 minutes to separate the same into the red blood cells and granulocyte layer as the lower layer and the mononuclear cell layer (lymphocyte layer), platelets and plasma as the upper layer. Thereafter, the plasma in the upper layer was inactivated in a water bath at 56°C for 30 minutes. The lymphocyte layer was collected in a 15-ml tube by a sterile pipette, and then centrifuged to remove the supernatant. The lymphocytes from which the supernatant has been removed were suspended in 10 ml of phosphate buffered solution (PBS) and washed. A portion of the suspension was used for cell count using a hemocytometer, and centrifuged again to collect only the lymphocytes. The total number of the harvested lymphocytes was measured to be $38 \times 10^6$ cells.

**(2) Step 2 - Immune Cell Culture (25T Flask)**

The lymphocytes isolated in step 1 were suspended in a culture medium (KBM 502) and then cultured using two 25T flasks (flask A and flask B) (hereinafter, culture in flask A is referred to as culture method A, and culture in flask B is referred to as culture method B). 50 to 300 $\mu$l of aldesleukin, IL-18, and human immunoglobulin were added to the cell suspension in each of flask A and flask B, and the immune cells were cultured in a 5% $CO_2$ incubator at 37°C for 1 to 2 days. The antibody and plasma in this step were added to the suspension on the day the culture was started.

**(3) Step 3 - Addition/Non-Addition of NK Cell-Activating Substances**

The suspension in each of flask A and flask B in step 2, in which the immune cells were being cultured, was suspended in fresh medium, and then aldesleukin, IL-18, and human immunoglobulin were added thereto in the same manner as in step 2.

**(4) Step 4 - Immune Cell Culture (75T Flask)**

The cells cultured in step 3 were transferred to a 75T flask, and the immune cells were cultured in a 5% $CO_2$ incubator at 37°C for 2 to 3 days. In this case, 50 to 300 $\mu$l of aldesleukin, IL-18, and human immunoglobulin were added to the cell suspension in each of flask A and flask B, and then the immune cells were transferred to the 75T flask and cultured in a 5% $CO_2$ incubator at 37°C for 1 to 2 days. The antibody and plasma in this step were added to the suspension on the day the culture was started.

### (5) Step 5 - Addition/Non-Addition of NK Cell-Activating Substances

The suspension in each of flask A and flask B in step 4, in which the immune cells were being cultured, was suspended in fresh medium, and then aldesleukin, IL-18, and human immunoglobulin were added thereto in the same manner as in step 4.

### (6) step 6 - Immune Cell Culture (175T Flask)

The cells cultured in step 5 were transferred to a 175T flask, and the immune cells were cultured in a 5% $CO_2$ incubator at 37°C for 2 to 3 days. In this case, 50 to 300 $\mu$l of aldesleukin, IL-18, and human immunoglobulin were added to the cell suspension in each of flask A and flask B, and flask B was treated with any one or more of panobinostat, trichostatin A, valproic acid, sodium butyrate, and suberoylanilide hydroxamic acid (SAHA) at a concentration of 100 to 500 $\mu$M.

### (7) step 7 - Addition/Non-Addition of NK Cell-Activating Substances

The suspension in each of flask A and flask B in step 6, in which the immune cells were being cultured, was suspended in fresh medium. Then, in the same manner as in step 6, aldesleukin, IL-18, and human immunoglobulin were added to flask A, and aldesleukin, IL-18, human immunoglobulin, and valproic acid were added to flask B.

### (8) Step 8 - Immune Cell Culture ($CO_2$ Permeable Bag)

The cell suspensions cultured in flask A and flask B in step 7 were transferred to 1,000-ml $CO_2$ permeable bags (bag A and bag B), respectively, and then the immune cells were cultured in a 5% $CO_2$ incubator at 37°C for 7 to 10 days. The plasma in this step was added on day 7 of culture. In addition, in this case, any one or more of panobinostat, trichostatin A, valproic acid, sodium butyrate, and suberoylanilide hydroxamic acid (SAHA) were added to bag B.

### (9) Step 9 - Immune Cell Harvesting

The cell suspensions according to culture methods A and B, which were contained in the $CO_2$ permeable bags in step 8, were transferred to centrifuge tubes and centrifuged at 2,500 rpm to harvest the cells. The supernatant was discarded, and the pellet containing the cells was washed twice with 250 ml of sterile saline, and then centrifuged to harvest the cells. The harvested cells were injected into a 100-ml pack containing sterile saline solution for injection, thereby completing the production of immune cells including NK cells with enhanced cytotoxicity.

### Experimental Example 1: Test for Analyzing Proportions of NK and NKT Cells

In order to analyze the proportions of the NK and NKT cells according to the present invention, non-treated cells and 1 x $10^6$/ml NK cells cultured in the presence of valproic acid, trichostatin A, SAHA, and panobinostat for 14 days were taken, and 0.5 $\mu$l each of CD3-APC, CD56-FITC, and CD16-PE-Cy7 were added thereto. Then, the cells were stained for 30 minutes at room temperature under a light-shielded condition. After staining, the cells were centrifuged at 1,200 rpm at 4°C for 3 minutes, the supernatant was removed, and the cells were suspended in PBS. Then, the proportions of NK and NKT cells were observed using flow cytometry.

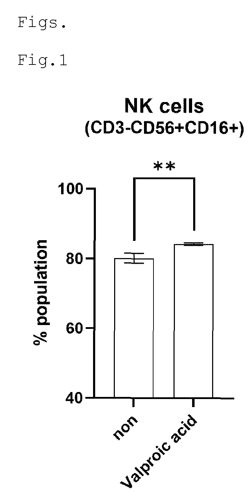

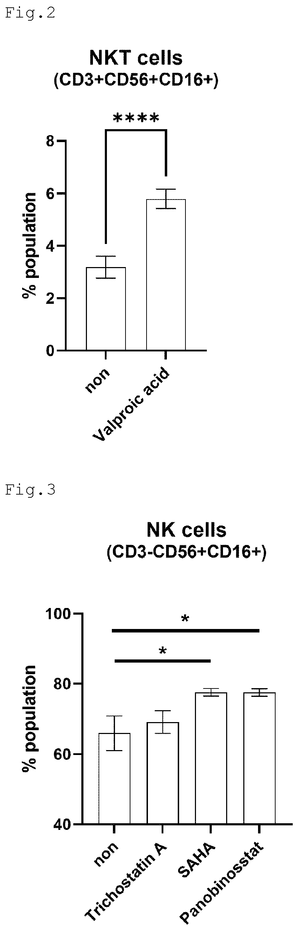

As a result, as shown in FIGS. 1 and 2, it was confirmed that the NK cell proportion and NKT cell proportion in the valproic acid-treated group significantly increased by 4% and 2.8%, respectively, compared to those in the non-treated group.

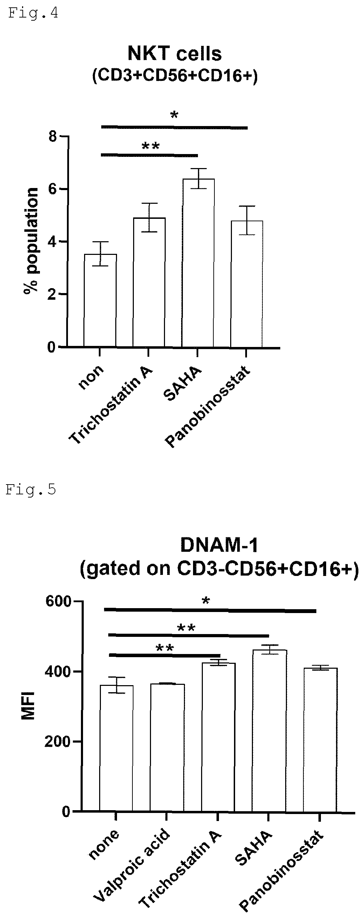

In addition, as shown in FIGS. 3 and 4, the NK cell proportion and NKT cell proportion in the trichostatin A-treated group increased by 3% and 1.4%, respectively, compared to those in the non-treated group, and the NK cell proportion and NKT cell proportion in the SAHA-treated group significantly increased by 11.6% and 2.9%, respectively, compared to those in the non-treated group.

It was confirmed that the NK cell proportion and NKT cell proportion in the panobinostat-treated group significantly increased by 11.6% and 1.32%, respectively, compared to those in the non-treated group.

Based on the above results, it was confirmed that NK cells grown in the presence of valproic acid, trichostatin A, SAHA, and panobinostat could have increased proportions of NK and NKT cells compared to the control group.

**Experimental Example 2: Test for Analyzing Expression Levels of Activated Receptors**

To analyze the expression levels of activated NK receptors in the non-treated group and the groups treated with valproic acid, trichostatin A, SAHA, and panobinostat, $1 \times 10^6$/ml NK cells cultured for 14 days were taken, 0.5 $\mu$l each of CD3-APC, CD56-FITC, CD16-PE-Cy7, NKG2D-PE, and DNAM-1-Percp-Cy5.5 were added thereto, and the cells were stained at room temperature for 30 minutes under a light-shielded condition. After staining, the cells were centrifuged at 1,200 rpm at 4°C for 3 minutes, the supernatant was removed, and the cells were suspended in PBS. Then, the expression levels of DNAM-1 and NKG2D in the cells were analyzed by flow cytometry.

As a result, as shown in FIG. 5, it was confirmed that the expression level of the DNAM-1 receptor in the NK cells cultured for 14 days significantly increased 1.2-fold in the trichostatin A-treated group, 1.3-fold in the SAHA-treated group, and 1.14-fold in the panobinostat-treated group.

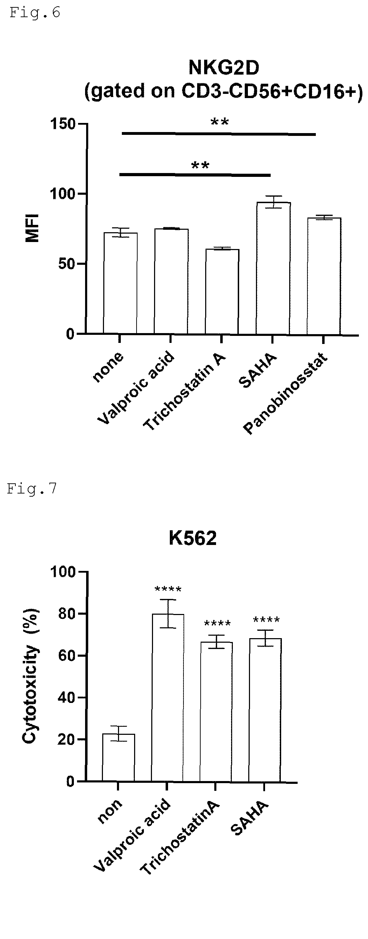

In addition, as shown in FIG. 6, it was confirmed that the expression level of the NKG2D receptor in the NK cells cultured for 14 days significantly increased 1.3-fold in the SAHA-treated group and about 1.2-fold in the panobinostat-treated group.

These results suggest that the NK cells grown in the presence of valproic acid, trichostatin A, SAHA, and panobinostat have increased expression levels of the activated receptors compared to those of the control group, and thus NK cells cultured in the presence of the above reagents can exhibit increased cytotoxicity.

**Experimental Example 3: Test for Evaluating Cytotoxicity of NK Cells against Cancer Cells**

Lung cancer cells (A549), breast cancer cells (MDA-MB231), hematological cancer cells (K562), epidermal cancer cells (A431), pancreatic cancer cells (Panc-1), and liver cancer cells (SK-Hep1) were stained with calcein-AM, homogenized in 2% FBS in phenol red-free RPMI media, and dispensed into v-bottom 96-well plates at $1 \times 10^4$ cells/well. The cultured NK cells were added at 40 or 100 times the number of the cancer cells, and the NK cells and the cancer cells were co-cultured in a 5% $CO_2$ incubator at 37°C incubator for 6 hours.

After completion of the co-culture, the cells were centrifuged at 1,000 rpm at 4°C for 3 minutes, and 100 $\mu$l of the supernatant was collected and transferred to a 96-well black plate. Fluorescence values at an excitation wavelength of 488 nm and an emission wavelength of 528 nm were measured using a fluorescence spectrophotometer, and cytotoxicity was calculated using the following equation:

$$\text{Cytotoxicity} \quad (\%) \quad = \quad \frac{Experimental(i) - TargetSpontaneouse(ii)}{TargetMaximum(iii) - TargetSpontaneous(ii)} \times 100$$

Experimental (i) = experimental - culture media background; Target Spontaneous (ii) = Target cell spontaneous calcein-am release - culture media background; Target Maximum (iii) = calcein AM release of target cells treated with 0.25% triton X-100 - culture media background.

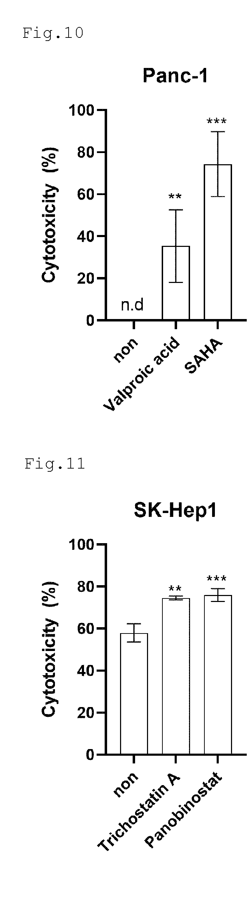

As a result, as shown in FIGS. 7 to 10 and FIG. 12, it was observed that the cytotoxicity of the valproic acid-treated group increased by 57% in the hematological cancer cell line (K562), 37% in the pancreatic cancer cell line (Panc-1), and 42% in the epidermal cancer cell line (A431), compared to that of the non-treated group.

As shown in FIGS. 7, 8, 9 and 11, it was observed that the cytotoxicity of the trichostatin A-treated group increased by 44% in the hematological cancer cell line (K562), 26% in the lung cancer cell line (A549), 14% in the breast cancer cell line (MDA-MB231), and 17% in the liver cancer cell line (SK-Hep1), compared to that of the non-treated group.

As shown in FIGS. 7, 8, 9, 10 and 12, it was observed that the cytotoxicity of the SAHA-treated group increased by 46% in the hematological cancer cell line (K562), 26% in the lung cancer cell line (A549), 18% in the breast cancer cell line (MDA-MB231), 39% in the epidermal cancer cell line (A431), and 74% in the pancreatic cancer cell line (Panc-1), compared to that of the non-treated group.

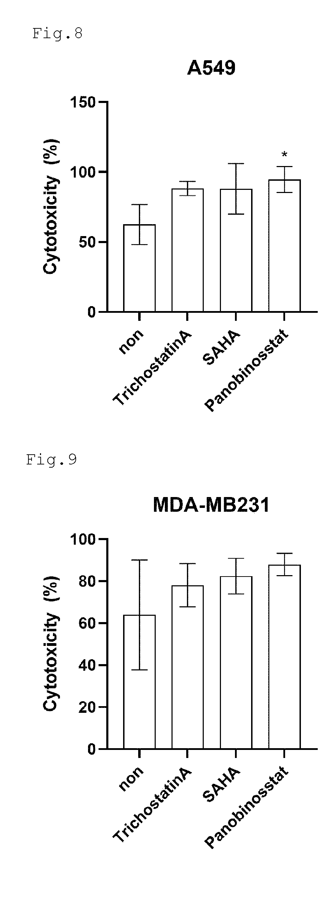

As shown in FIGS. 8, 9 and 11, it was observed that the cytotoxicity of the panobinostat-treated group increased by 33% in the lung cancer cell line (A549), 24% in the breast cancer cell line (MDA-MB231), and 18% in the liver cancer cell line (SK-Hep1), compared to that of the non-treated group.

Based on the above results, it can be seen that the NK cells cultured in the presence of valproic acid, trichostatin A, SAHA, and panobinostat have increased cytotoxicity against most solid cancers and hematological cancers compared to the control group.

**Experimental Example 4: Test for Analyzing Amount of Interferon Gamma (IFN-γ) Secreted by NK Cells**

In order to analyze the amount of INF-γ secreted by the NK cells when co-culturing the NK cells produced by the production method according to one embodiment of the present invention with cancer cells, non-treated cells or the NK cells cultured in the presence of valproic acid, trichostatin A, SAHA, and panobinostat for 14 days were co-cultured with cancer cells in 96 well-plates for 6 hours.

Specifically, after 6 hours of co-culture, the 96 wells were centrifuged at 1,000 rpm at 4°C for 3 minutes, and the supernatant was collected and measured for the amount of INF-r secreted.

The IFN-γ measurement method followed the directions for the human IFN-gamma quantikine ELISA kit (R&D Systems).

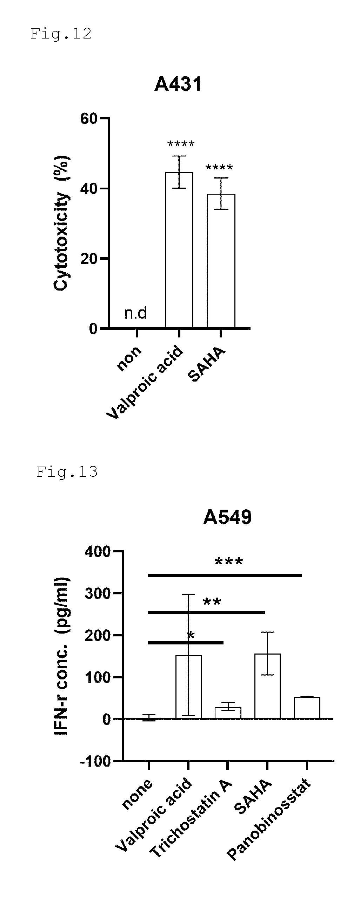

As a result, as shown in FIG. 13, it can be seen that, when the NK cells were co-cultured with the lung cancer cell line A549, the amount of IFN-γ secreted by the NK cells significantly increased 42-fold in the valproic acid-treated group, 8.3-fold in the trichostatin A-treated group, 43.1-fold in the SAHA-treated group, and 14.5-fold in the panobinostat-treated group compared to that in the control group.

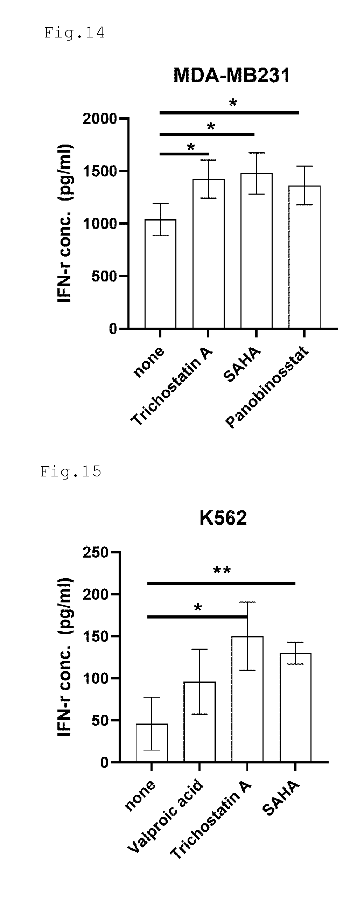

As shown in FIG. 14, it can be seen that, when the NK cells were co-cultured with the breast cancer cell line MDA-MB231, the amount of IFN-γ secreted by the NK cells significantly increased 1.4-fold in the trichostatin A-treated group, 1.4-fold in the SAHA-treated group, and 1.3-fold in the panobinostat-treated group compared to that in the control group.

As shown in FIG. 15, it can be seen that, when the NK cells were co-cultured with the hematological cancer cell line K562, the amount of IFN-γ secreted by the NK cells significantly increased 2-fold in the valproic acid-treated group, 3.3-fold in the trichostatin A-treated group, and 2.8-fold in the SAHA-treated group compared to that in the control group.

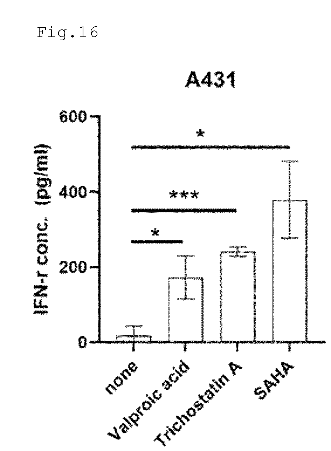

As shown in FIG. 16, it can be seen that, when the NK cells were co-cultured with the epidermal cancer cell line A431, the amount of IFN-γ secreted by the NK cells significantly increased 9.5-fold in the valproic acid-treated group, 13.2-fold in the trichostatin A-treated group, and 20.8-fold in the SAHA-treated group compared to that in the control group.

Based on the above results, it was confirmed that the NK cells cultured in the presence of valproic acid, trichostatin A, SAHA, and panobinostat had a cytotoxic effect against most solid cancer and hematological cancer cells compared to the control group.

So far, the present invention has been described with reference to the embodiments. Those of ordinary skill in the art to which the present invention pertains will appreciate that the present invention may be embodied in modified forms without departing from the essential characteristics of the present invention. Therefore, the disclosed embodiments should be considered from an illustrative point of view, not from a restrictive point of view. The scope of the present invention is defined by the claims rather than the foregoing description, and all differences within the scope equivalent thereto should be construed as being included in the present invention.

**Claims**

1. A method for producing immune cells with enhanced cytotoxicity, comprising:

   step (a) of repeating a process of adding any one or more of aldesleukin, IL-15, IL-18, anti-CD56 antibody, human immunoglobulin, and plasma to lymphocytes and culturing the lymphocytes, 1 to 7 times;
   step (b) of further adding any one or more of panobinostat, trichostatin A, valproic acid, sodium butyrate, and suberoylanilide hydroxamic acid (SAHA) during any one or more culturing processes in the step of repeating; and
   step (c) of harvesting the cultured lymphocytes.

2. The method of claim 1, wherein step (a) comprises adding any one or more of anti-CD56 antibody and human immunoglobulin and any one or more of aldesleukin, IL-15, IL-18 and plasma.

3. The method of claim 1, wherein the anti-CD56 antibody is added in an amount of 0.5 to 5 μl, and the human immunoglobulin is added in an amount of 5 to 500 μl.

4. The method of claim 1, wherein the valproic acid is added at a concentration of 50 to 5000 μM.

5. A method for producing immune cells with enhanced cytotoxicity, comprising:

   step (a) of performing any one or more of the following in culture of lymphocytes: ① adding any two or more of aldesleukin, IL-15, IL-18, anti-CD56 antibody, human immunoglobulin, and plasma; and ② adding any one or more of panobinostat, trichostatin A, valproic acid, sodium butyrate, and SAHA; and
   step (b) of harvesting the lymphocytes cultured in step (a).

6.   The method of claim 5, further comprising, after step (a), step (a-1) of performing any one or more of the following: ① adding any one or more of aldesleukin, IL-15, IL-18, anti-CD56 antibody, human immunoglobulin, and plasma to the lymphocytes; and ② adding any one or more of panobinostat, trichostatin A, valproic acid, sodium butyrate, and SAHA to the lymphocytes.

7.   The method of claim 6, further comprising, after step (a-1), step (a-2) of performing any one or more of the following: ① adding any two or more of aldesleukin, IL-15, IL-18, anti-CD56 antibody, human immunoglobulin, and plasma to the lymphocytes; and ② adding any one or more of panobinostat, trichostatin A, valproic acid, sodium butyrate, and SAHA to the lymphocytes.

8.   The method of claim 7, further comprising, after step (a-2), step (a-3) of performing any one or more of the following: ① adding any two or more of aldesleukin, IL-15, IL-18, anti-CD56 antibody, human immunoglobulin, and plasma to the lymphocytes; and ② adding any one or more of panobinostat, trichostatin A, valproic acid, sodium butyrate, and SAHA to the lymphocytes.

9.   The method of claim 8, further comprising, after step (a-3), step (a-4) of performing any one or more of the following: ① adding any two or more of aldesleukin, IL-15, IL-18, anti-CD56 antibody, human immunoglobulin, and plasma to the lymphocytes; and ② adding any one or more of panobinostat, trichostatin A, valproic acid, sodium butyrate, and SAHA to the lymphocytes.

10.   The method of claim 9, further comprising, after step (a-4), step (a-5) of performing any one or more of the following: ① adding any two or more of aldesleukin, IL-15, IL-18, anti-CD56 antibody, human immunoglobulin, and plasma to the lymphocytes; and ② adding any one or more of panobinostat, trichostatin A, valproic acid, sodium butyrate, and SAHA to the lymphocytes.

11.   The method of any one of claims 5, 7 and 9, wherein the addition of any two or more of aldesleukin, IL-15, IL-18, anti-CD56 antibody, human immunoglobulin, and plasma is treatment with any one or more of anti-CD56 antibody and human immunoglobulin and any one or more of aldesleukin, IL-15, IL-18, and plasma.

12.   The method of any one of claims 5, 7 and 9, wherein the anti-CD56 antibody is added in an amount of 0.5 to 5 $\mu$l, and the human immunoglobulin is added in an amount of 5 to 500 $\mu$l.

13.   The method of any one of claims 5, 7 and 9, wherein the valproic acid is added at a concentration of 50 to 5,000 $\mu$M.

Figs.

Fig.1

**NK cells**
**(CD3-CD56+CD16+)**

Fig.2

**NKT cells**
**(CD3+CD56+CD16+)**

Fig.3

**NK cells**
**(CD3-CD56+CD16+)**

Fig.4

**NKT cells**
**(CD3+CD56+CD16+)**

Fig.5

**DNAM-1**
**(gated on CD3-CD56+CD16+)**

Fig.6

**NKG2D
(gated on CD3-CD56+CD16+)**

Fig.7

**K562**

Fig.8

**A549**

Fig.9

**MDA-MB231**

Fig.10

**Panc-1**

Fig.11

**SK-Hep1**

Fig.12

**A431**

Fig.13

**A549**

Fig.14

**MDA-MB231**

Fig.15

**K562**

Fig.16

**EP 4 560 013 A1**

**INTERNATIONAL SEARCH REPORT**

International application No.

**PCT/KR2023/002414**

| A. | CLASSIFICATION OF SUBJECT MATTER |
|---|---|

**C12N 5/0783**(2010.01)i

According to International Patent Classification (IPC) or to both national classification and IPC

| B. | FIELDS SEARCHED |
|---|---|

Minimum documentation searched (classification system followed by classification symbols)

C12N 5/0783(2010.01); A23L 33/10(2016.01); A61K 35/17(2015.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 면역세포(immune cell), 림프구(lymphocyte), 자연살해세포(natural killer cell, NK cell), 확장(expansion), 인터루킨(interleukin), 항-CD 항체(anti-CD antibody), 히스톤 탈아세틸화 효소 저해제(histone deacetylase inhibitor, HDAC inhibitor)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2022-0036287 A (TS BIO CO., LTD.) 22 March 2022 (2022-03-22)<br>See paragraphs [0009]-[0073]. | 1-13 |
| Y | KR 10-2019-0131239 A (KOREA UNIVERSITY RESEARCH AND BUSINESS FOUNDATION) 26 November 2019 (2019-11-26)<br>See paragraphs [0019]-[0032]. | 1-13 |
| A | KR 10-2020-0061240 A (CHA UNIVERSITY INDUSTRY-ACADEMIC COOPERATION FOUNDATION et al.) 02 June 2020 (2020-06-02)<br>See entire document. | 1-13 |
| A | KR 10-2017-0007692 A (KOREA UNIVERSITY RESEARCH AND BUSINESS FOUNDATION) 19 January 2017 (2017-01-19)<br>See entire document. | 1-13 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 June 2023** | **07 June 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/002414** |

**C.     DOCUMENTS CONSIDERED TO BE RELEVANT**

| Category* | Citation of document, with indication, where appropriate, of the relevant passages | Relevant to claim No. |
| --- | --- | --- |
| A | KR 10-2019-0060412 A (SUNGKWANG MEDICAL FOUNDATION) 03 June 2019 (2019-06-03)<br>     See claim 1. | 1-13 |

Form PCT/ISA/210 (second sheet) (July 2022)

**INTERNATIONAL SEARCH REPORT**
Information on patent family members

International application No.

**PCT/KR2023/002414**

| Patent document cited in search report | | | Publication date (day/month/year) | Patent family member(s) | | | Publication date (day/month/year) |
|---|---|---|---|---|---|---|---|
| KR | 10-2022-0036287 | A | 22 March 2022 | WO | 2022-060056 | A1 | 24 March 2022 |
| KR | 10-2019-0131239 | A | 26 November 2019 | CN | 112368371 | A | 12 February 2021 |
| | | | | EP | 3812458 | A1 | 28 April 2021 |
| | | | | KR | 10-2069704 | B1 | 23 January 2020 |
| | | | | US | 2021-0198627 | A1 | 01 July 2021 |
| | | | | WO | 2019-221463 | A1 | 21 November 2019 |
| KR | 10-2020-0061240 | A | 02 June 2020 | | None | | |
| KR | 10-2017-0007692 | A | 19 January 2017 | KR | 10-1851270 | B1 | 25 April 2018 |
| | | | | US | 11110126 | B2 | 07 September 2021 |
| | | | | US | 2017-0119865 | A1 | 04 May 2017 |
| | | | | US | 2019-0183995 | A1 | 20 June 2019 |
| KR | 10-2019-0060412 | A | 03 June 2019 | JP | 2021-502796 | A | 04 February 2021 |
| | | | | JP | 7119076 | B2 | 16 August 2022 |
| | | | | KR | 10-2265437 | B1 | 15 June 2021 |
| | | | | US | 2021-0095249 | A1 | 01 April 2021 |
| | | | | WO | 2019-103436 | A2 | 31 May 2019 |
| | | | | WO | 2019-103436 | A3 | 18 July 2019 |
| | | | | WO | 2019-103436 | A9 | 22 August 2019 |

Form PCT/ISA/210 (patent family annex) (July 2022)